# EUROPEAN PATENT SPECIFICATION

(11) **EP 3 492 021 B1**
(45) Date of publication and mention of the grant of the patent: **13.01.2021**
(21) Application number: 17204709.4
(22) Date of filing: 30.11.2017
(51) Int. Cl.: A61B 17/04, A61B 17/06, A61B 17/00

(54) **INSERT HOLDING DEVICE, SUTURE HOLDING DEVICE AND METHOD FOR CONNECTING A SUTURE TO AN INSERT FOR INSERTION INTO A BONE**
EINSATZHALTEVORRICHTUNG, NAHTHALTEVORRICHTUNG UND VERFAHREN ZUM VERBINDEN EINER NAHT MIT EINEM EINSATZ ZUM EINSETZEN IN EINEN KNOCHEN
DISPOSITIF DE MAINTIEN D'INSERT, DISPOSITIF DE RETENUE DE SUTURE ET PROCÉDÉ DE CONNEXION D'UNE SUTURE À UN INSERT DESTINÉ À ÊTRE INSÉRÉ DANS UN OS

(43) Date of publication of application: 05.06.2019
(73) Proprietor: ZuriMED Technologies AG, 8008 Zürich (CH)
(72) Inventor: SNEDEKER, Jess, 8008 Zürich (CH); BACHMANN, Elias, 8706 Meilen (CH); LI, Xiang, 8126 Zumikon (CH); BEELER, Silvan, 8704 Herrliberg (CH); GÖTSCHI, Tobias, 8048 Zürich (CH)
(74) Representative: Schulz Junghans Patentanwälte PartGmbB

(56) References cited:
- EP-A2- 2 777 512
- WO-A1-2017/023883
- GB-A- 2 092 253
- KR-B1- 101 728 634
- US-A- 4 613 265
- US-A- 5 078 730
- US-A- 5 715 942
- US-A- 6 080 184

## Description

The invention relates to an insert holding device and a suture holding device for connecting a suture to an insert for insertion into a bone, a kit of parts comprising the insert holding device and the suture holding device, and a method for connecting a suture to an insert for insertion into a bone. In particular, the suture and the insert may be comprised in an implant for ligament repair in a human or animal joint, more particularly anterior cruciate ligament repair in a human or animal knee.

Implants for ligament repair comprising a flexible graft (e.g. an autograft from a patient hamstring) connected to at least one osteoconductive element (OCE) are known from the prior art (i.e. WO 2016/113142 A1). In addition, so-called bone-tendon-bone (BTB) grafts have been described in the prior art.

In such implants, the flexible graft replaces the torn or damaged ligament (e.g. the anterior cruciate ligament, ACL), and the osteoconductive element or bone graft is inserted into boreholes of associated bones (such as the tibia and/or femur in case of ACL repair).

Therein, the purpose of the insert is to fix the implant in the bone and to facilitate ingrowth of bone cells leading to increased long-term stability of the implant.

In addition, so-called cortical fixation devices (CFD) are typically used to attach the implant on the outside of the borehole of the associated bone to apply sufficient tension to the flexible graft for replacing the function of the native ligament. Typical CFDs comprise a plate member to be positioned outside of the borehole, wherein sutures extending through the bone tunnel connect the plate member to the osteoconductive element or bone graft.

In clinical practice, attaching sutures to an insert for insertion into a bone (such as an OCE or a bone graft), in particular to connect the insert to a CFD or a flexible graft, is challenging for the surgeon, particularly since small components are handled. However, positioning the insert in the implant correctly is of great importance for achieving good biological performance.

Prior art document WO 2017/023883 discloses a suture keeper including an elongate, generally planar body comprising a first slot and a second slot each defining a cantilevered tab, the tabs being configured to hold a suture anchor between them. The body further comprises pairs of holes flanking one of the slots and notches on opposite sides of the body configured to receive sutures to be connected to the suture anchor.

Therefore, the objective of the present invention is to provide a means to facilitate attachment of sutures to an insert for insertion into a bone.

This objective is attained by the subject matter of independent claim 1 relating to an insert holding device, independent claim 3 relating to a suture holding device, dependent claim 6 relating to a kit of parts, and independent claim 7 relating to a method for connecting a suture to an insert for insertion into a bone.

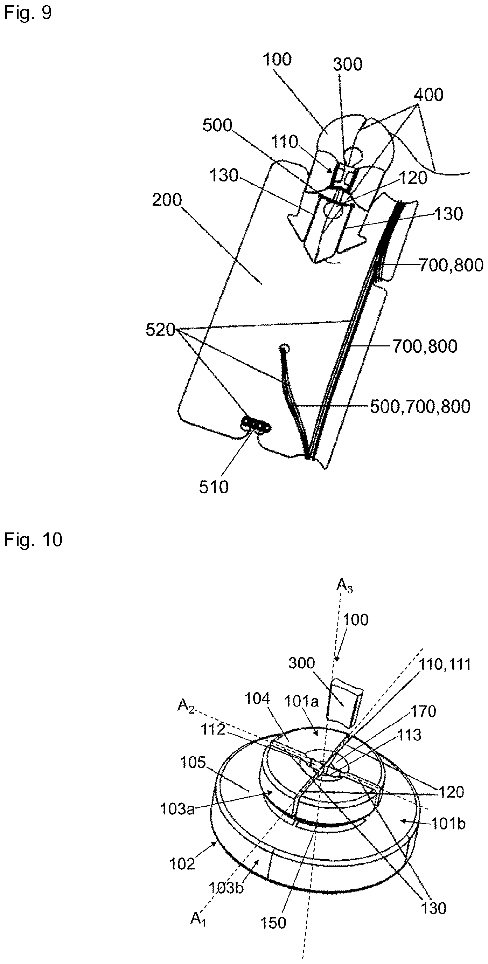

Embodiments of the present invention are described in dependent claims 2, 4 to 5 and 8 and are described hereafter. Embodiments according to the invention are depicted in figures 10-12.

A first aspect of the invention relates to an insert holding device for connecting a suture to an insert for insertion into a bone, comprising a holding structure configured to releasably hold an insert for insertion into a bone, at least one first recess for receiving a first suture, and at least one second recess for receiving a looped second suture, wherein the at least one first recess and the at least one second recess are configured such that the first suture can be laid around the second suture and the insert, thereby connecting the second suture to the insert when the insert is held by the holding structure.

The insert holding device comprises a first surface, a second surface facing away from the first surface, and a circumferential third surface connecting the first surface and the second surface, wherein the at least one first recess and the at least one second recess are open towards the first surface and closed towards the second surface, and wherein the first recess extends along a first axis and the second recess extends along a second axis perpendicular to the first axis.

The at least one first recess is configured such that the first suture can be arranged around the insert when the insert is held by the holding structure.

By means of the holding structure and the first and second recess, the insert and the first and second sutures are pre-arranged, such that a surgeon may easily attach the second suture to the insert by laying the first suture around the insert and second suture and e.g. tying a knot of the first suture.

In the context of the present specification, the term 'insert' describes any component suitable to be inserted into a bone of a human or animal, in particular components comprising an osteoconductive and/or osteoinductive material. This includes bone grafts, such as xenografts and allografts, in other words natural bone material, as well as artificial osteoconductive and/or osteoinductive materials, such as e.g. tricalcium phosphate (TCP). Therein the term 'osteoinductive' describes a material which is adapted to serve as a scaffold for new bone growth that is perpetuated by the native bone. The term 'osteoconductive' describes a material which is adapted to stimulate osteoprogenitor cells to differentiate into osteoblast, thereby facilitating new bone formation.

In the context of the present specification, the term 'suture' describes any elongated member which is suitable to be used for connecting components, particularly by sewing or knotting, to be implanted into the human or animal body.

The insert holding device can be manufactured from any suitable material, such as a metal or a polymer.

In particular, the insert is adapted for use in an implant for anterior cruciate ligament repair, wherein the implant further comprises a flexible graft, particularly replacing the anterior cruciate ligament. More particularly, the implant further comprises a cortical fixation device comprising a plate member for applying tension to the flexible graft.

Due to its anatomical location, the anterior cruciate ligament (ACL) is subjected to potentially extreme forces during sports and other physical activities. Rupture of the ACL has been counted as the most frequent and severe of ligament injuries. Although several surgical options, including hamstring graft approach, bone patella tendon bone graft, etc. for ACL reconstruction have been practiced for the restoration of knee joint function, several unavoidable drawbacks exist, such as donor site morbidity, ligament laxity, mechanical mismatch, etc.

Clinically implemented graft fixation methods largely rely on tendon to bone ingrowth to provide long term ligament stability, with many requiring bone ingrowth to a non-osteoconductive graft. The non-osteoconductive nature of the graft material often critically limits the success of ACL repair. Failure of timely bone ingrowth can lead to "bone tunnel expansion", and ultimately a failed reconstruction that may necessitate a revision surgery with large cost and burden to the patient.

To minimize bone tunnel expansion and to achieve an optimized attachment of a graft within the bone tunnel, an osteo-conductive element (OCE) can be used between bone tunnel and graft, resulting in a so-called "bony bridge". Such an OCE has been described in patent application WO 2016/113142 A1.

In certain embodiments, the at least one first recess of the insert holding device is configured such that the first suture can be arranged in a straight line. In certain embodiments, the at least one second recess is configured such that the second suture can be arranged in a straight line.

The at least one first recess is configured such that the first suture can be arranged along a first axis when the first suture is received in the at least one first recess, wherein the at least one second recess is configured such that the second suture can be arranged along a second axis, when the second suture is received in the at least one second recess, wherein the first axis is non-parallel to the second axis and wherein the first axis is arranged at an angle of 90° in respect of the second axis.

In certain embodiments, the holding structure of the insert holding device comprises a slot for receiving the insert, a first wall and a second wall opposing the first wall, wherein the slot is delimited by the first wall and the second wall, and wherein the first wall and the second wall are adapted to releasably hold the insert within the slot by static friction. In other words, the insert is held in the slot by a press-fit. Alternatively, any other suitable means for holding the insert in place can be used as a holding structure.

In particular, the slot is open along the first axis, such that the insert can be slid in the slot along the first axis.

In certain embodiments, the first wall and/or the second wall comprises a flexible material having a Young's modulus of 10000 MPa or less, particularly 5000 MPa or less, more particularly 2000 MPa or less, most particularly 1800 MPa, according to DIN EN ISO 178:2013-09. Thus, due to the flexibility of the material, static friction with the insert is generated when the first and/or second wall is deformed by the insert in the slot.

In certain embodiments, the first wall and/or the second wall comprises or consists of polypropylene.

In certain embodiments the insert holding device comprises a force-generating element, for example a spring, such as a leaf spring, adapted to exert a force on the first and/or second wall. Thereby, static friction with the insert is generated when the insert is positioned in the slot.

In an embodiment according to the invention, the insert holding device comprises a first recess, wherein the first recess extends along the first axis. In other words, the at least one first recess consists of the first recess (there is only one first recess).

In an embodiment according to the invention, the insert holding device comprises two second recesses. In other words: the at least one second recess comprises or is constituted by two second recesses. In certain embodiments, the two second recesses extend along the first axis.

In certain embodiments, the second recesses each comprise a width of 1 mm or less, particularly 0,5 mm or less. Therein, in particular, the width is measured along the second axis. Due to the small width, a certain amount of force has to be applied to move a surgical suture of typical width through the second recess, such that the suture is prevented from slipping out of the second recess.

In certain embodiments, the at least one second recess comprises a respective holding section for receiving the second suture and a respective entry section for moving the second suture towards the respective holding section, particularly from an opening in the third surface, wherein the respective holding section comprises a width, particularly along the second axis, which is larger than a width, particularly along the second axis, of the respective entry section. In particular, this prevents moving of the second suture back into the entry section.

In certain embodiments, the entry section comprises a width of 0,5 mm or less, particularly 0,2 mm or less.

The at least one first recess and the at least one second recess are open towards the first surface and closed towards the second surface. Therein, in particular, the at least one first recess is adapted to receive the first suture from the first surface, and the at least one second recess is adapted to receive the second suture from the first surface, wherein more particularly the holding structure of the insert holding device is adapted to hold the insert above the first suture and the second suture, such that the first suture can be arranged around the insert and the second suture, thereby connecting the second suture to the insert. Therein, in particular, the insert holding device comprises a first recess and a second recess, in other words, the at least one first recess consists of a (single) first recess and the at least one second recess consists of a (single) second recess.

In certain embodiments, the slot of the holding structure of the insert holding device is formed by a blind hole which is open towards the first surface. In certain embodiments, the slot is positioned at an intersection between the at least one first recess and the at least one second recess, particularly between the first recess and the second recess.

In an embodiment according to the invention, the insert holding device comprises a groove for inserting the second suture, wherein the groove extends along the third surface of the insert holding device in a circumferential direction, and wherein the at least one second recess is connected to, particularly extends into, the groove.

In certain embodiments, the insert holding device comprises an upper portion and a lower portion, wherein the upper portion comprises the at least one first recess and the at least one second recess, particularly the first recess and the second recess, wherein particularly the third axis extends along a third axis which is perpendicular to the first axis and the second axis.

In certain embodiments, the upper portion comprises a respective first surface and a respective circumferential third surface, and the lower portion comprise a respective first surface, a second surface opposing the respective first surface, and a respective third circumferential surface connecting the first surface and the second surface of the lower portion. Therein, in particular, the second surface of the lower portion is the same as the second surface of the insert holding device.

In certain embodiments, the groove extending along the third surface of the insert holding device in a circumferential direction is positioned on the third surface of the upper portion, in particular adjacent to the first surface of the lower portion.

In certain embodiments, the upper portion and the lower portion are arranged along a third axis, wherein the upper portion comprises a first maximum extension perpendicular to the third axis, in particular a first diameter, and the second section comprises a second maximum extension perpendicular to the third axis, in particular a second diameter, wherein the first maximum extension is smaller than the second maximum extension.

In certain embodiments, the insert holding device comprises a circular cross-section in respect of the third axis.

In certain embodiments, the upper portion and the lower portion comprise a circular cross-section in respect of the third axis. Therein, in particular, the first maximum extension of the upper portion perpendicular to the third axis is the first diameter of the upper portion, and the second maximum extension of the lower portion perpendicular to the third axis is the second diameter of the lower portion.

In certain embodiments, the insert holding device is adapted to be mechanically connected to a suture holding device for holding at least the second suture, particularly according to the second aspect of the invention.

A second aspect of the invention relates to a suture holding device for connecting a suture to an insert for insertion into a bone comprising a first recess for inserting a flexible graft, a holding structure for positioning a looped second suture across the first recess, wherein the first recess is configured such that an insert for insertion into a bone held by the holding structure of an insert holding device, in particular according to the first aspect of the invention, can be positioned in the first recess and a first suture can be laid around the second suture and the insert, thereby connecting the second suture to the insert.

The suture holding device is formed from a flat sheet of material arranged in a plane defined by a first axis and a second axis, wherein the suture holding device comprises a front surface and a back surface opposing the front surface, and wherein said first recess is a circular-shaped central first recess, and wherein said holding structure comprises a first through-hole and a second through-hole arranged along the second axis in line with the first recess.

The suture holding device comprises a first notch and a second notch in opposing edges, wherein the first notch and the second notch are arranged along the first axis in line with the first recess.

The suture holding device can be combined with the insert holding device according to the first aspect by placing the suture holding device on top of the insert holding device such that at least an upper portion of the insert holding device is positioned in the first recess, such that the first axis of the insert holding device is parallel to the first axis of the suture holding device, and the second axis of the insert holding device is parallel to the second axis of the suture holding device.

The suture holding device is configured to be assembled with an insert holding device according to the first aspect of the invention, such that the first suture can be laid around the second suture and the insert, thereby connecting the second suture to the insert, when the insert is held by the holding structure of the insert holding device and the suture holding device and the insert holding device are assembled.

In certain embodiments, the first recess of the suture holding device is configured such that a flexible graft (in particular a tendon graft, e.g. from a hamstring) can be passed through the first recess in order to connect the flexible graft to the second suture.

In certain embodiments, the suture holding device comprises a further holding structure for positioning a first suture across the first recess.

In certain embodiments, the suture holding device comprises at least one groove for arranging at least one further suture (for example a passing suture and/or a flipping suture of a cortical fixation device) on the suture holding device.

In certain embodiments, the suture holding device comprises at least one hole for arranging at least one further suture (for example a passing suture and/or a flipping suture of a cortical fixation device) on the suture holding device.

A third aspect of the invention relates to a kit of parts comprising an insert holding device according to the first aspect of the invention and a suture holding device according to the second aspect of the invention, wherein the first recess of the suture holding device is configured such that an insert for insertion into a bone held by the holding structure of the insert holding device can be positioned in or adjacent to the first recess of the suture holding device, and a first suture can be laid around a second suture and the insert, thereby connecting the second suture to the insert, wherein the second suture is positioned across the first recess of the suture holding device by the holding structure of the suture holding device.

A fourth aspect of the invention relates to a method for connecting a suture to an insert for insertion into a bone, wherein an insert holding device according to the first aspect of the invention, is provided, and wherein an insert for insertion into a bone is received by a holding structure of the insert holding device, such that the holding structure releasably holds the insert, and wherein a first suture is received by at least one first recess of the insert holding device, and wherein a looped second suture is received by at least one second recess of the insert holding device, and wherein the first suture is laid around the second suture and the insert held by the holding structure of the insert holding device, thereby connecting the second suture to the insert.

The second suture is held by a holding structure of a suture holding device according to the second aspect, wherein the second suture is positioned across a first recess of the suture holding device, and wherein the insert held by the holding structure of the insert holding device is positioned in said first recess of the suture holding device, and wherein the first suture is laid around the second suture held by the holding structure of the suture holding device and the insert held by the holding structure of the insert holding device, thereby connecting the second suture to the insert.

In certain embodiments, the insert is subsequently removed from the holding structure of the insert holding device.

In certain embodiments, a flexible graft is connected to the insert, particularly after removing the insert from the holding structure of the insert holding device. Therein, in particular, the flexible graft is connected to the second suture, thereby connecting the flexible graft to the insert. For example, the flexible graft can be folded around the second suture and optionally stitched together at at least one stitching position.

In certain embodiments, the insert is first received by the holding structure of the insert holding device, wherein the second suture is subsequently received by the at least one second recess of the insert holding device, and wherein the first suture is subsequently received by the at least one first recess of the insert holding device.

In certain embodiments, the insert is first received by the holding structure of the insert holding device, wherein the first suture is subsequently received by the at least one first recess of the insert holding device, and wherein the second suture is subsequently received by the at least one second recess of the insert holding device.

In certain embodiments, the first suture is first received by the at least one first recess of the insert holding device, wherein the second suture is subsequently received by the at least one second recess of the insert holding device, and wherein the insert is subsequently received by the holding structure of the insert holding device.

In certain embodiments, the first suture is tied into a knot around the second suture and the insert, thereby connecting the second suture to the insert.

In certain embodiments, the insert holding device is removed from the suture holding device, wherein a flexible graft is inserted into the first recess of the suture holding device after removing the insert holding device from the suture holding device, and wherein the flexible graft is connected to the second suture, thereby connecting the flexible graft to the insert. For example, the flexible graft can be folded around the second suture and optionally stitched together at at least one stitching position.

In certain embodiments, the insert, the first suture, the second suture, and the flexible graft are removed from the suture holding device after connecting the flexible graft to the second suture, wherein the flexible graft is pulled, and wherein particularly the suture holding device is bent. In particular, the plate member is removed from the suture holding device after connecting the flexible graft to the second suture.

The invention and other aspects of this disclosure are further described by the following examples and figures, from which additional embodiments may be derived. Therein
- Fig. 1: shows a schematic overview of anterior cruciate ligament repair according to the prior art;
- Fig. 2: shows a schematic drawing of a cortical fixation device according to the prior art;
- Fig. 3: shows a perspective view of the insert holding device according to an aspect of the disclosure;
- Fig. 4: shows a perspective view of the insert holding device according to an aspect of the disclosure, an insert for insertion into a bone, a first suture and a second suture;
- Fig. 5: shows a top view of an intermediate product for generating an insert holding device according to an aspect of the disclosure;
- Fig. 6: shows a perspective view of a suture holding device according to an aspect of the disclosure with a second suture and a cortical fixation device arranged on the suture holding device;
- Fig. 7: shows a top view of the front surface of the suture holding device according to an aspect of the disclosure and a cortical fixation device arranged on the suture holding device;
- Fig. 8: shows a top view of the back surface of the suture holding device according to an aspect of the disclosure and a cortical fixation device arranged on the suture holding device;
- Fig. 9: shows a perspective view of an assembly between an insert holding device and a suture holding device according to an aspect of the disclosure;
- Fig. 10: shows a perspective view of an embodiment of the insert holding device according to the invention;
- Fig. 11: shows a schematic of a method for connecting a suture to an insert using the insert holding device according to an embodiment;
- Fig. 12: shows a perspective view of an embodiment of the suture holding device according to the invention.

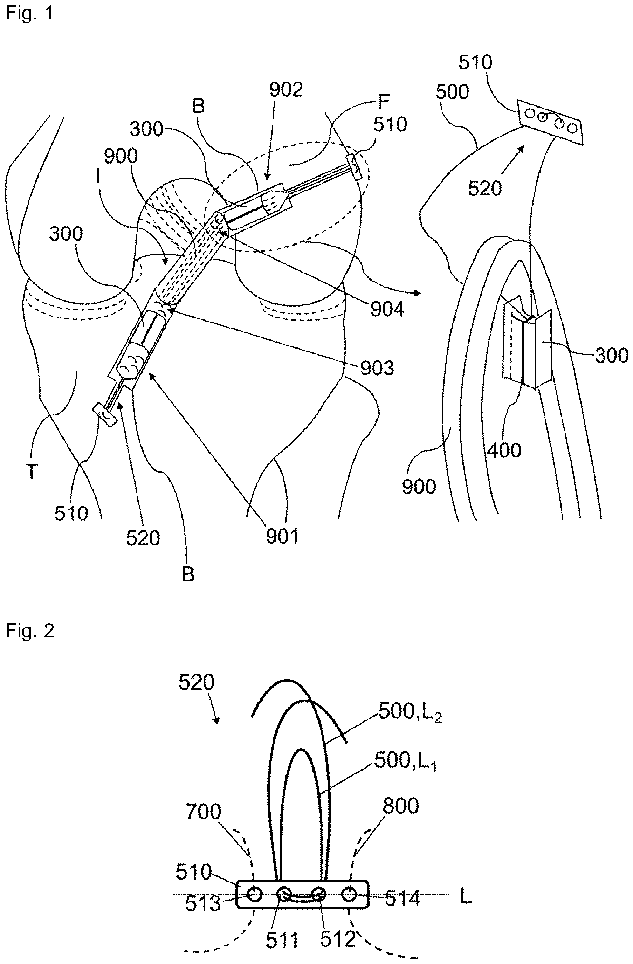

Fig. 1 shows a schematic overview of an example of anterior cruciate ligament repair in a human knee by means of an implant I according to the prior art. The implant I comprises a flexible graft 900 connected at a first end 901 and a second end 902 to a respective insert 300. The inserts 300 (as well as a part of the flexible graft 900) are positioned in boreholes B of the tibia T and femur F, respectively. Furthermore, each insert 300 is connected to a respective looped second suture 500 of a respective cortical fixation device 520 by means of a first suture 400 laid around the respective insert 300 and the respective second suture 500. The cortical fixation devices 520 further comprise respective plate members 510 which are connected to the looped second sutures 500. The plate members 510 are positioned on the outside of the respective boreholes B of the tibia T and femur F.

The implant I thus extends from the outside of the tibia T bone through the borehole B of the tibia T, the joint space and the borehole B of the femur F to the outside of the femur F bone, wherein the flexible graft 900 replaces the native anterior cruciate ligament. Tension can be applied to the flexible graft 900 by pulling on the second sutures 500 between the plate members 510 and the inserts 300, wherein the plate members 510 prevent the ends of the second sutures 500 from being pulled into the respective borehole B when tension is applied to the flexible graft 900.

Typically, the flexible graft 900 is an autograft comprising segments cut from a patient's hamstring tendon.

In particular, the insert 300 comprises an osteoconductive and/or osteoinductive material, such that ingrowth of bone cells into the implant I occurs, improving the stability of the implant I. Such an insert 300 is also termed "osteoconductive element". Alternatively, the insert 300 may be constituted by a bone block, preferably an autograft. In this case, the resulting implant I is termed "bone-tendon-bone autograft".

A schematic drawing of a typical plate member 510 of a cortical fixation device 520 for fixing an implant I (such as shown in Fig. 1) is depicted in Fig. 2. The plate member 510 comprises a first hole 511 and a second hole 512 for receiving a second suture 500, for example to connect the plate member 510 to the insert 300. As shown in Fig. 2, the second suture 500 is arranged in a closed loop L₁ and an open loop L₂.

Furthermore, the plate member 510 comprises a third hole 513 for receiving a pulling suture 700 and a fourth hole 514 for receiving a flipping suture 800. The third hole 513 and the fourth hole 514 are positioned adjacent to opposing ends of the plate member 510 along a longitudinal axis L of the plate member 510. The pulling suture 700 can be used to pull the plate member 510 through a borehole B of the femur F and/or tibia T bone, wherein the longitudinal axis L of the plate member 510 is parallel to the borehole B. After the plate member 510 has reached the outside of the borehole B, the plate member 510 can be rotated by pulling the flipping suture 800, such that the longitudinal axis L is perpendicular to the borehole B. In this configuration, the plate member 510 prevents the end of the second suture 500 from being pulled into the borehole B when tension is applied to the flexible graft 900 (see also Fig. 1).

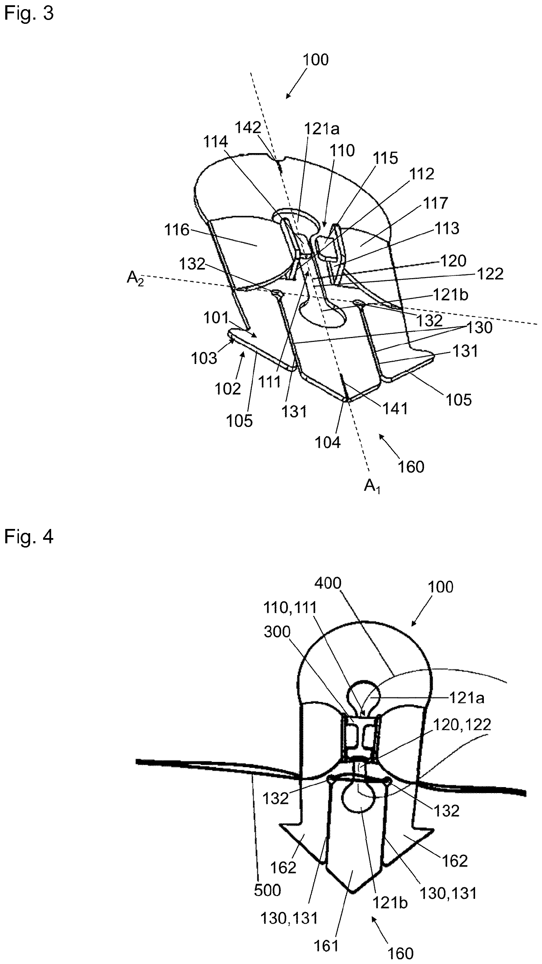

Fig. 3 shows a first example of an insert holding device 100 according to an aspect of the disclosure, which is configured to assist in connecting a second suture 500 of a cortical fixation device 520 to an insert 300 (i.e. an osteoconductive element) of an implant for anterior cruciate ligament repair. The insert holding device 100 comprises a flat sheet of material having a first surface 101 and a second surface 102 opposing the first surface 101, wherein the first surface 101 and the second surface 102 are connected by a third, circumferential, surface 103 formed by an edge of the sheet of material.

Furthermore, the insert holding device 100 comprises a holding structure 110 for receiving an insert 300, wherein the holding structure 110 comprises a first wall 112, a second wall 113 opposing the first wall 112, and a third wall 114 and a fourth wall 115 extending parallel to the first surface 101 and perpendicular to the first wall 112and the second wall 113. The first wall 112 is connected to the third surface 103 (edge) of the insert holding device 100 by means of a first strut 116, and the second wall 113 is connected to the third surface 103 by means of a second strut 117 opposite the first strut 116.

The first wall 112, the second wall 113, the third wall 114 and the fourth wall 115 form a slot 111 for receiving the insert 300, wherein the first and the second wall 112,113 form side walls of the slot 111 and the third and the fourth wall 114,115 form a roof of the slot 111. The slot 111 is open along a first axis A₁ of the insert holding device 100, such that an insert 300 can be slid in the slot 111 along the first axis A₁.

In particular, the first wall 112 and the second wall 113 are formed from a flexible material, and the distance between the first wall 112 and the second wall 113 is chosen such that an insert 300 of appropriate dimensions is held in the slot 111 by static friction (press-fit) between the first wall 112, the second wall 113 and the insert 300 when no external force is applied, but can be easily removed from the slot 111 by applying a force along the first axis A₁ (for instance by hand). Optionally, the third and fourth wall 114,115 forming the roof of the slot 111 can also be adapted to generate static friction to hold the insert 300 in the slot 111.

Alternatively, the insert holding device 100 may comprise at least one force-generating element (e.g. a leaf spring) adapted to exert a force on an insert 300 in the slot 111, wherein the force acts perpendicular to the first axis A₁. In this manner, a press-fit of the insert 300 in the slot 111 may be achieved with first and second walls 112,113 from a less flexible material.

As shown in Fig.3, the insert holding device 100 further comprises a first recess 120 formed by a through-hole extending along the first axis A₁ between the first wall 112 and the second wall 113 of the holding structure 110. The first recess 120 comprises opposing first and second end sections 121a, 121b connected by a middle section 122. Therein, the width of the end sections 121a, 121b (the maximum extension perpendicular to the first axis A₁) is greater than the width of the middle section 122. In other words, the first recess 120 is dumbbell-shaped.

Furthermore, the insert holding device 100 comprises a first notch 141 and a second notch 142 opposing the first notch 142, wherein the first notch 141 and the second notch 142 are arranged along the first axis A₁ in line with the first recess 120. The first and second notch 141,142 are configured to receive a first suture 400 and hold the first suture 400 in place when the first suture 400 is arranged along the first recess 120. The first suture 400 can be received in the first and second notch 141,142 by inserting the first suture 400 from the third surface 103 of the insert holding device 100, in other words from its edge. In particular, the first and second notch 141,142 are narrow enough, such that a certain force has to be applied to insert the first suture 400 into the first and second notch 141,142, wherein the first suture 400 is held by static friction once inserted into the first and second notch 141,142.

In addition, the insert holding device 100 according to the example shown in Fig. 3 to Fig. 5 comprises two second recesses 130 formed by parallel notches extending along the first axis A₁ on either side of the first recess 120. The second recesses 130 each comprise a respective entry section 131 and a respective holding section 132. Therein, the entry sections 131 are open towards the third surface 103 (edge) and separate a pointed end 160 of the insert holding device 100 into a middle segment 161 and two side segments 162. A second suture 500 may be inserted from the third surface 103 into the entry sections 131 and moved along the entry sections 131 into the holding sections 132. The holding sections 132 are arranged along a second axis A₂, which is perpendicular to the first axis A₁, and wherein the second axis A₂ intersects the first recess 120. Therefore, when the second suture 500 is arranged in a straight line from one of the holding sections 132 across the first surface 101 of the insert holding device 100 to the other holding section 132, the second suture 500 extends above the first recess 120.

The width (maximum extension measured perpendicular to the first axis A₁) of the entry sections is smaller than the width of the respective holding sections 132. In particular, the width of the entry sections 131 is such that a certain amount of force has to be applied to insert the second suture 500 into the entry sections 131 (see Fig. 4). In this manner, the second suture 500 can be prevented from moving back from the holding sections 132 into the entry sections 131.

In particular, as illustrated in Fig. 4, for attaching the first suture 500 to the insert 300, the insert 300 is first inserted into the slot 111 of the holding structure 110 of the insert holding device 100. Subsequently, the first suture 400 is passed through the first end section 121a of the first recess 120 from the first surface 101 to the second surface 102, passed below the insert 300 held by the holding structure 110, and passed through the second end section 121b of the first recess 120 back from the second surface 102 to the first surface 101. In case the optional first and second notch 141,142 is provided, the first suture 400 can subsequently be fixed by inserting the first suture 400 into the first and second notch 141,142 (see Fig. 9). Passing the first suture 400 through the first recess 120 is facilitated by the increased width of the end sections 121a, 121b of the first recess 120. Next, a second suture 500 is inserted through the entry sections 131 into the holding sections 132 of the second recesses 130, such that the second suture 500 extends above the first suture 400 across the first recess 120. Therein, in particular, the second suture 500 is connected to a plate member 510 of a cortical fixation device 520. Finally, the first suture 400 is laid around the insert 300 held by the holding structure 110 and around the second suture 500 held by the second recesses 130, straightened and tied into a knot, thereby connecting the insert 300 to the second suture 500. Alternatively to tying of a knot, the first suture 400 can be fixed by other means, such as e.g. gluing.

Of course, in case the first suture 400 has been fixed by insertion into the first and second notch 141,142, the first suture 400 has to be removed from the first and second notch 141,142 prior to laying the first suture 400 around the insert 300 and the second suture 500 and tying the knot.

The insert holding device 100 facilitates connecting the insert 300 to the second suture 500, since the insert 300, the second suture 500, and (optionally) also the first suture 400 can be secured on the insert holding device 100 in an arrangement allowing fast and easy connection by means of the first suture 400.

After joining of the insert 300 and the second suture 500, the insert holding device 100 can be removed and the insert 300 can be connected to a flexible graft 900 to generate an implant I, in particular for anterior cruciate ligament repair, in a manner known from the prior art (see Fig. 1).

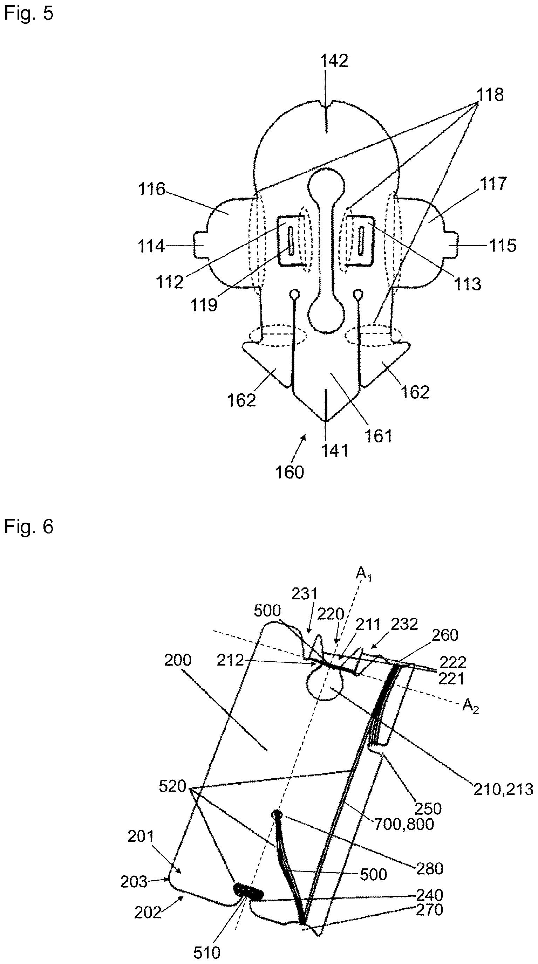

Fig. 5 illustrates a method of manufacture of the insert holding device 100 shown in Fig. 3 and Fig. 4. In this embodiment, the device 100 consists of a flat sheet of material, into which folding lines 118 are introduced (e.g. by indenting or heat treatment). The folding lines 118 are indicated by dashed lines, whereas cutting lines are indicated by solid lines. In order to generate the first wall 112 and the second wall 113 (see Fig. 3 and Fig. 4), two essentially rectangular segments of the sheet are cut out at three sides and a folding line 118 is generated at a fourth side extending along the first axis A₁ on the inside of the respective rectangular segment. Additionally, respective slits 119 are cut out in the center of the rectangular segments. The rectangular segments are then folded inside towards the first surface 101 in order to generate the first and second wall 112,113.

Further fold 118 lines are generated to delimit segments constituting the first and second struts 116,117, and the third and fourth wall 114,115. The segments constituting the struts 116,117 are then folded inside towards the first surface 101, the segment constituting the third wall 114 is inserted into the slit 119 of the first wall 112, and the segment constituting the fourth wall 115 is inserted into the slit 119 of the second wall 113.

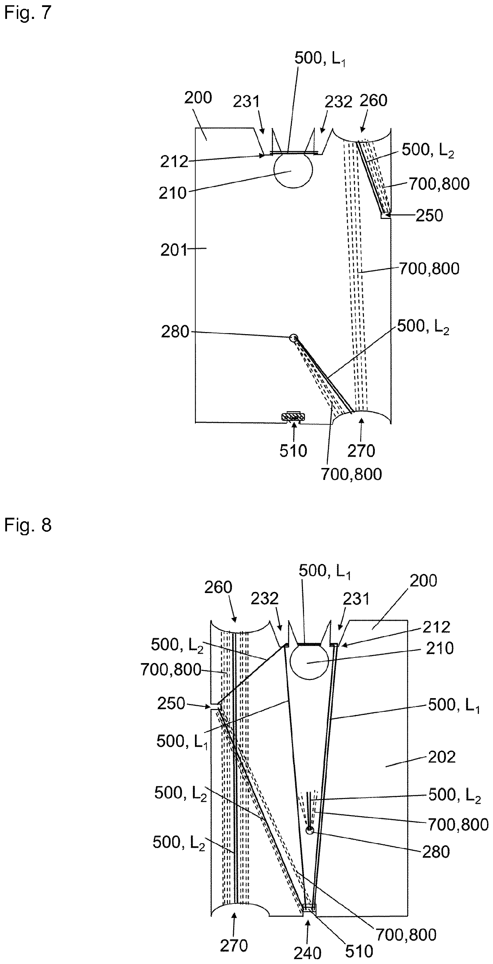

The fold lines 118 on the side segments 162 of the pointed end 160 can be used to fold the side segments upwards in order to facilitate joining of the insert holding device 100 with the suture holding device 200 described below (see Fig. 7). Folding the side segments 162 upwards is advantageous to prevent locking the insert holding device 100 on the suture holding device 200.

Optionally, the above-described insert holding device 100 can be combined with a suture holding device 200 according to an aspect of the disclosure as depicted in Fig. 6 and Fig. 7.

As shown in Fig. 6, the suture holding device 200 consists of a flat sheet of material extending in a plane defined by a first axis A₁ and a second axis A₂ which is perpendicular to the first axis A₁. The suture holding device 200 has a front surface 201, a back surface 202 opposing the front surface 201, and a circumferential edge 203 connecting the front surface 201 and the back surface 202.

Furthermore, the suture holding device 200 comprises a first recess 210 comprising a slot section 211 and a circular section 213 connected at a neck 212. The first recess 210 is positioned adjacent to a holding structure 220 for arranging a second suture 500 across the first recess 210 along the second axis A₂.

The holding structure 220 comprises a first protrusion 221 and a second protrusion 222 opposite the first protrusion 221, wherein the slot section 211 of the first recess 210 is positioned between the first and the second protrusion 221,222.The protrusions 221,222 each comprise a triangular shape, wherein the protrusions 221 each comprise an inner edge adjacent to the slot section 211, wherein the inner edges are arranged at an acute angle in respect of the first axis A₁, such that the width of the slot section 211 (measured along the second axis A₂) decreases towards the circular section 213 of the first recess 210, thereby forming a neck 212 at the narrowest position.

A second recess 231 is positioned adjacent to the first protrusion 221 opposite the slot section 211, and a third recess 232 is positioned adjacent to the second protrusion 222 opposite the slot section 211.

Fig. 6 further shows a part of a looped second suture 500 held by the holding structure 220. The second suture 500 extends from the back surface 202 through the second recess 231 to the front surface 201, across the first protrusion 221, the neck 212 of the first recess 210 and the second protrusion 222, and back to the back surface 202 through the third recess 232. To obtain the configuration shown in Fig. 6, the second suture 500 can be inserted into the second and third recess 231,232 from the edge 203 of the suture holding device 200 along the first axis A₁. This has the advantage that a second suture 500 arranged in a closed loop (e.g. as part of a cortical fixation device 520) can be received by the holding structure 220 without opening the loop.

Furthermore, the suture holding device 200 comprises a first groove 240 for receiving a plate member 510, particularly a plate member 510 of a cortical fixation device 520 as described above and depicted in Fig. 2. The first groove 240 of the suture holding device 200 is positioned opposite the first holding structure 220 along the first axis A₁. When the plate member 510 is connected to the second suture 500, a closed loop of the second suture 500 extending between the holding structure 220 and the plate member 510 in the first groove 240 on the back surface 202 of the suture holding device 200 can be tightened and thereby fixed on the suture holding device 200.

Moreover, according to the embodiment shown in Fig. 6, the suture holding device 200 comprises a second groove 250, a third groove 260, a fourth groove 270, and an opening 280. The third and the fourth groove 260,270 are positioned opposite to each other along the first axis A₁ on a side of the suture holding device 200 in respect of the holding structure 220 The second groove 250 is positioned between the third and the fourth groove 260,270 and extends along the second axis A₂ on the side of the suture holding device 200. The opening 280 is positioned between the first recess 210 and the first groove 240.

A cortical fixation device 520 (see Fig. 2) comprising a second suture 500 (also termed graft suture) for connecting a plate member 510 to a flexible graft 900, a pulling suture 700 for pulling the plate member 510 through a borehole B of a bone, and a flipping suture 800 for flipping the plate member 510 outside of the borehole B can be arranged on the suture holding device 200 according to the embodiment shown in Fig. 6 as described below and illustrated in Fig. 7 and Fig. 8.

As shown in Fig. 7 and Fig. 8, the second suture 500 is arranged on a plate member 510 in a closed loop L₁ and an open loop L₂ as shown in Fig. 2, wherein the second suture 500 extends twice through the first hole 511 and the second hole 512 of the plate member 510. The pulling suture 700 is arranged in the third hole 513 of the plate member 510 and the flipping suture is arranged in the fourth hole 514 of the plate member 510. In order to fix the cortical fixation device 520 on the suture holding device 500, the closed loop L₁ is inserted in the second and third recess 231,232 of the holding structure 220 of the suture holding device 200, such that the second suture 500 extends across the neck 212 of the first recess 210, and the plate member 510 is inserted into the first groove 240 of the suture holding device 200, such that the plate member 510 is arranged on the front side 201 of the suture holding device 200 and the closed loop L₁ extends from the first hole 511 of the plate member 510 to the second recess 231 on the back surface 202 of the suture holding device 200, across the first protrusion 221, the neck 212 of the first recess 210 and the second protrusion 222 on the front surface 201 of the suture holding device 200, and from the third recess 232 back to the second hole 512 of the plate member 510 on the back surface 202 of the suture holding device 200. The closed looped L₁ can be tightened by pulling the ends of the open loop L₂ in order to tightly fix the closed loop L₁ on the suture holding device 200.

One end of the open loop L₂ is then arranged from the plate member 510 to the third recess 232 on the back surface 202, across the neck 212 of the first recess 210 to the second recess 231 on the front surface 201, and from the second recess 231 to the second groove 250 on the back surface 202 of the suture holding device 200. The other end of the open loop L₂ is arranged from the plate member 510 to the second groove 250 on the back surface 202 of the suture holding device 200. Subsequently, the first and the second end of the open loop L₂ are arranged from the second groove 250 to the third groove 260 on the front surface 201, from the third groove 260 to the fourth groove 270 on the back surface 202, from the fourth groove 270 to the opening 280 on the front surface 201, and through the opening 280 to the second surface 202 of the suture holding device 200.

The pulling suture 700 and the flipping suture 800 are arranged from the plate member 510 to the second groove 250 on the back surface 202, from the second groove 250 to the third groove 260 on the front surface 201, from the third groove 260 to the fourth groove 270 on the back surface 202, from the fourth groove 270 to the third groove 260 on the front surface 201, again from the third groove 260 to the fourth groove 270 on the back surface 202, from the fourth groove 270 to the opening 280 on the front surface 201 and through the opening 280 to the back surface 202 of the suture holding device 200.

The final configuration of the sutures on the front surface 201 of the suture holding device 200 is depicted in Fig. 7, and the final configuration of the sutures on the back surface 202 of the suture holding device 200 is depicted in Fig. 8.

Fig. 9 illustrates how the suture holding device 200 according to Fig. 6 to Fig. 8 can be combined with an insert holding device 100 according to the aspect of the disclosure depicted in Fig. 1 to Fig. 4.

The insert 300 is first received by the holding structure 110 of the insert holding device 100 (e.g. inserted into the slot 111), and the first suture 400 is received by the first recess 120 of the insert holding device 100 as described above. In detail, the first suture 400 is passed from the first surface 101 of the insert holding device 100 through the first end section 121a of the first recess 120 to the second surface 102, passed below the insert 300 towards the second end section 121b of the first recess 120, and passed through the second end section 121b back to the first surface 101. Optionally, as shown in Fig. 9, the first suture 400 is inserted into the first notch 141 and the second notch 142 (see Fig. 5) to fix and secure the first suture 400 on the insert holding device 100.

Next, the second suture 500 is arranged on the holding structure 220 of the suture holding device 200 as described above (Fig. 6 to Fig. 8), such that the second suture 500 extends above the neck 212 of the first recess 210 of the suture holding device 200.

Subsequently, the insert holding device 100 is assembled with the suture holding device 200 as shown in Fig. 9. Therein, the pointed end 160 (see Fig. 4) of the insert holding device 100 is slid on the front surface 201 of the suture holding device 200, wherein the first axis A₁ of the insert holding device 100 (see Fig. 4) is parallel to the first axis A₁ of the suture holding device 200 (see Fig. 6), such that the middle segment 161 of the pointed end 160 is arranged below the second suture 500 on the holding structure 210 of the suture holding device 200. In this manner, the second suture 500 is received by the second recesses 130 of the insert holding device 100.

The pointed end 160 facilitates positioning of the middle segment 161 below the second suture 500 on the holding structure 210 of the suture holding device 200.

In addition, the arrow shape of the side segments 162 indicates the direction of joining the insert holding device 100 with the suture holding device 200.

When the insert holding device 100 and the suture holding device 200 have been joined as shown in Fig. 9, the second suture 500 lays on top of the first suture 400, such that the second suture 500 can be connected to the insert 300 by laying the first suture 400 around the second suture 500 and the insert 300 and e.g. tying a knot into the first suture 400. After this step, the insert holding device 100, the suture holding device 200, and the insert 300 connected to the second suture 500 (and the cortical fixation device 520 attached thereto) can be disassembled, and the cortical fixation device 520 can e.g. be connected to the flexible graft 900.

After this step, the insert holding device 100 can be firstly removed from the suture holding device 200. Therein, the insert 300 is moved out of the slot 111, and stays attached to the suture 500. Then, a flexible graft 900 (see Fig. 1) can be put through the first recess 210 (particularly through the circular section 213 of the first recess), thereby inserting the flexible graft 900 into the first loop L₁ of the second suture 500 (see Fig. 2), and folded to attach the flexible graft 900 to the insert 300. Optionally, the flexible graft 900 can be stitched together at a first stitching position 903 and a second stitching position 904 of the flexible graft 900 to secure the flexible graft 900 on the insert 300.

Finally, the whole implant comprising the insert 300, the first suture 400, the second suture 500, the plate member 510, and the flexible graft 900 can be removed from the suture holding device 200 by pulling the flexible graft 900 and slightly bending the suture holding device 200 on the back surface 202.

Fig. 10 shows an embodiment of the insert holding device 100 according to the invention. The insert holding device 100 comprises an upper portion 104 and a lower portion 105 arranged along a third axis A₃. Both the upper portion 104 and the lower portion 105 have a cylindrical shape with a circular cross-section in respect of the third axis A₃, wherein the diameter of the upper portion 104 is smaller than the diameter of the lower portion 105. The upper portion 104 comprises a first surface 101a perpendicular to the third axis A₃ and a circumferential third surface 103a, and the lower portion 105 comprises a first surface 101b perpendicular to the third axis A₃, a second surface 102 opposing the first surface 101b, and a circumferential third surface 103b.

The insert holding device 100 comprises a holding structure 110 comprising a slot 111 extending along a second axis A₂ perpendicular to the third axis A₃ on the first surface 101a of the upper portion 104 delimited by a first wall 112 and a second wall 113. Therein, the material of the walls 112,113 and the distance between the first wall 112 and the second wall 113 are chosen such that an insert 300 can be held in position by static friction between the first wall 112, the second wall 113 and the insert 300 when inserted into the slot 111, and easily removed from the slot (press-fit). For instance, the first wall 112 and the second wall 113 may comprise a flexible material which is compressed when the insert 300 is inserted into the slot 111.

Furthermore, the holding device 100 comprises a first recess 120 and a second recess 130 on the first surface 101a of the upper portion 104, wherein the first recess 120 extends along a first axis A₁ perpendicular to the second axis A₂ and the third axis A₃, and the second recess 130 extends along the second axis A₂. In other words, the first and the second recess 120 are formed by a cross-shaped slot on the upper portion 104.

In the example depicted in Fig. 10, the slot 111 of the holding structure 110 overlaps with and forms part of the second recess 130. Alternatively, the slot 111 may form part of the first recess 120. Along the third axis A₃, the first recess 120 and the second recess 130 extend towards the first surface 101b of the lower portion 105. In other words, the first recess 120 and the second recess 130 comprise the same height as the upper portion, and the first surface 10b of the lower portion 105 forms the bottom of the first and second recess 120,130. A conical recess 170 (or a recess having any other shape, such as a rectangular milled recess) may be provided around the slot 111 to facilitate insertion of the insert 300 into the slot 111. The third surface 103a of the upper portion 104 further comprises a groove 150 which extends circumferentially in respect of the third axis A₃.

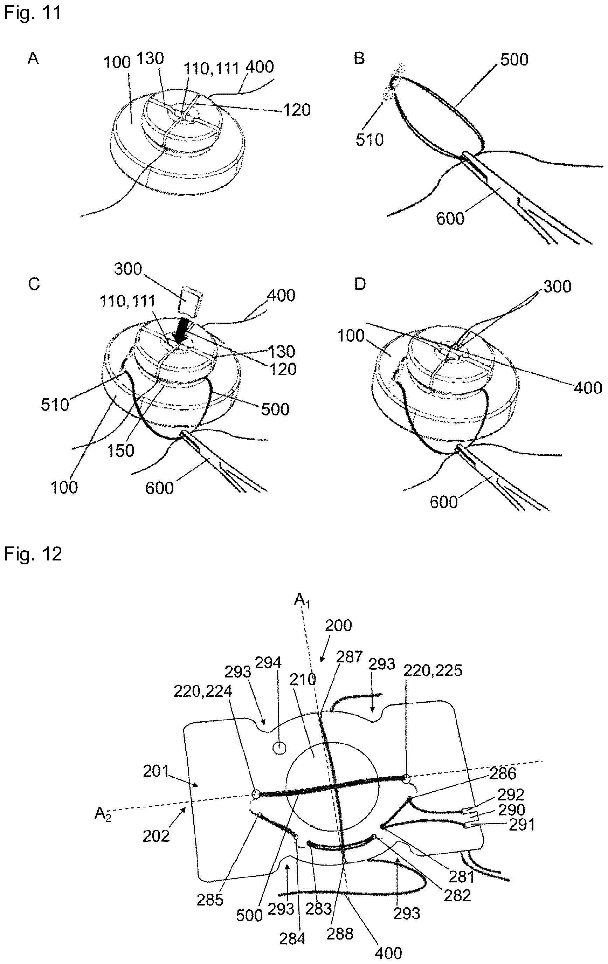

Fig. 11 schematically depicts an example of the use of the insert holding device 100 according to the embodiment shown in Fig. 10 for connecting a second suture 500 (e.g. of a cortical fixation device 520, see Fig. 2) to an insert 300 (e.g. an osteoconductive element or a bone graft). In a first step (Fig. 11A), a first suture 400 is placed in the first recess 120 of the insert holding device 100. According to a second step (Fig. 11B), a looped second suture 500 connected to a plate member 510 of a cortical fixation device 520 (see Fig. 2) is gripped by a surgical tool 600. As shown in Fig. 11C, the loop of the second suture 500 is then inserted into the second recess 130 of the insert holding device 100, and the loop is partially inserted into the circumferential groove 150 which facilitates holding the second suture 500 in place by means of the tool 600. Subsequently, as depicted in Fig. 12C, the insert 300 is placed in the slot 111 above the first suture 400 and the second suture 500 and held in position by the holding structure 110. Finally, as shown in Fig. 12D, the first suture 400 is tied into a knot, thereby connecting the second suture 500 to the insert 300.

Fig. 12 depicts an embodiment of the suture holding device 200 according to the invention which can be used together with the insert holding device 100 according to Fig. 10. The suture holding device 200 is formed from a flat sheet of material arranged in a plane defined by a first axis A₁ and a second axis A₂, wherein the suture holding device 200 comprises a front surface 201 and a back surface 202 opposing the front surface 201. Furthermore, the suture holding device 200 comprises a circular-shaped central first recess 210 and a holding structure 220 comprising a first through-hole 224 and a second through-hole 225 arranged along the second axis A₂ in line with the first recess 210.

The suture holding device 200 further comprises a first hole 281, a second hole 282, a third hole 283, a fourth hole 284, and a fifth hole 285 arranged essentially along a semi-circle around the first recess 210. Moreover, the suture holding device 200 comprises a first notch 287 and a second notch 288 in opposing edges, wherein the first and the second notch 287,288 are arranged along the first axis A₁ in line with the first recess 210. In addition, a third notch 291 and a fourth notch 292 are provided in a further edge of the suture holding device 200, wherein a flap 290 is positioned between the third notch 291 and the fourth notch 292.

A second suture 500 can be arranged on the front surface 201 of the suture holding device 200 from the third notch 291 to the first hole 281, through the first hole 281 to the back surface 202 of the suture holding device 200, on the back surface 202 from the first hole 281 to the second hole 282, through the second hole 282 to the front surface 201, on the front surface 201 from the second hole 282 to the third hole 283, through the third hole 283 to the back surface 202, on the back surface 202 to the fourth hole 284, through the fourth hole 284 to the front surface 201, on the front surface 201 to the fifth hole 285, through the fifth hole 285 to the back surface 202, on the back surface 202 to the first through-hole 224 of the holding structure 220, through the first through-hole 224 to the front surface 201, on the front surface 201 across the first recess 210 to the second through-hole 225 of the holding structure 220, through the second through-hole 225 to the back surface 202, on the back surface 202 to the sixth hole 286, through the sixth hole 286 to the front surface 201, and on the front surface 201 back to the first hole 281, resulting in a closed loop L₁ of the second suture 500. The second suture 500 can then be passed along in parallel to the closed loop L₁ back to the sixth hole 286 and on the front surface 201 from the sixth hole 286 to the second notch 292, resulting in an additional open loop L₂ of the second suture 500 (see Fig. 2).

This results in the second suture 500 being fixed on the suture holding device 200 and arranged across the first recess 210 along the second axis A₂. Of course, instead of the first to sixth hole 281-286 and the third and fourth notch 291,292, other suitable means of fixing the second suture 500 on the suture holding device 200 may be used.

A first suture 400 may be arranged between the first notch 287 and the second notch 288 across the first recess 210, in particular above the second suture 500.

Four second recesses 293 on opposing edges of the suture holding device 200 may match the surface of the insert holding device 100 (Fig. 10, Fig. 11 A) to facilitate handling of the sutures 400,500 during placement on the insert holding device 100 (Fig. 10, Fig. 11 A).

The suture holding device 20 according to Fig. 12 can be combined with the insert holding device 100 according to Fig. 10 by placing the suture holding device 200 on top of the insert holding device 100 such that at least the upper portion 104 of the insert holding device 100 is positioned in the first recess 210, such that the first axis A₁ of the insert holding device 100 is parallel to the first axis A₁ of the suture holding device 200, and the second axis A₂ of the insert holding device 100 is parallel to the second axis A₂ of the suture holding device 200.

In this manner, the first suture 400 may be easily be inserted into the first recess 120 of the insert holding device 100, and the second suture 500 may be easily inserted into the second recess 130 of the insert holding device 100. Subsequently, the insert 300 can be placed into the slot 111 of the holding structure 110 of the insert holding device 100, and the first suture 400 can be laid around the insert 300 and the second suture 500 (and e.g. tied into a knot), thereby connecting the second suture 500 to the insert 300.

Optionally, the diameter of the first recess 210 may be chosen according to the diameter of the upper portion 104 of the insert holding device 100, such that only the upper portion 104 is inserted into the first recess 210, and the suture holding device 200 rests on the first surface 101b of the lower portion 105 of the insert holding device 100. This facilitates assembly of the insert holding device 100 and the suture holding device 200.

A position reference hole 294 on the suture holding device 200 may be aligned with a marking (not shown) of the insert holding device 100 during assembly of the parts in order to position the first axis A₁ of the insert holding device 100 parallel to the first axis A₁ of the suture holding device 200.

### List of reference numerals

| | |
|---|---|
| Insert holding device | 100 |
| First surface | 101, 101a, 101b |
| Second surface | 102 |
| Third surface | 103, 103a, 103b |
| Upper portion | 104 |
| Lower portion | 105 |
| Holding structure of the insert holding device | 110 |
| Slot | 111 |
| First wall | 112 |
| Second wall | 113 |
| Third wall | 114 |
| Fourth wall | 115 |
| First strut | 116 |
| Second strut | 117 |
| Folding line | 118 |
| Slit | 119 |
| First recess | 120 |
| First end section | 121a |
| Second end section | 121b |
| Middle section | 122 |
| Second recess | 130 |
| Entry section | 131 |
| Holding section | 132 |
| First notch | 141 |
| Second notch | 142 |
| Groove of the insert holding device | 150 |
| Pointed end | 160 |
| Middle segment | 161 |
| Side segment | 162 |
| Conical recess | 170 |
| Suture holding device | 200 |
| Front surface | 201 |
| Back surface | 202 |
| Edge | 203 |
| First recess of the suture holding device | 210 |
| Slot section | 211 |
| Neck | 212 |
| Circular section | 213 |
| Holding structure of the suture holding device | 220 |
| First protrusion | 221 |
| Second protrusion | 222 |
| First through-hole | 224 |
| Second through-hole | 225 |
| Second recess | 231 |
| Third recess | 232 |
| First groove | 240 |
| Second groove | 250 |
| Third groove | 260 |
| Fourth groove | 270 |
| Opening | 280 |
| First hole | 281 |
| Second hole | 282 |
| Third hole | 283 |
| Fourth hole | 284 |
| Fifth hole | 285 |
| Sixth hole | 286 |
| First notch of the suture holding device | 287 |
| Second notch of the suture holding device | 288 |
| Flap | 290 |
| Third notch of the suture holding device | 291 |
| Fourth notch of the suture holding device | 292 |
| Second recess of the suture holding device | 293 |
| Position reference hole | 294 |
| Insert | 300 |
| First suture | 400 |
| Second suture | 500 |
| Plate member | 510 |
| First hole | 511 |
| Second hole | 512 |
| Third hole | 513 |
| Fourth hole | 514 |
| Cortical fixation device | 520 |
| Tool | 600 |
| Pulling suture | 700 |
| Flipping suture | 800 |
| Flexible graft | 900 |
| First end | 901 |
| Second end | 902 |
| First stitching position | 903 |
| Second stitching position | 904 |
| First axis | A₁ |
| Second axis | A₂ |
| Third axis | A₃ |
| B | Borehole |
| I | Implant |
| F | Femur |
| T | Tibia |
| L | Longitudinal axis |
| L₁ | Closed loop |
| L₂ | Open loop |

## Claims

1. Insert holding device (100) for connecting a suture (500) to an insert (300) for insertion into a bone, comprising
a. a holding structure (110) configured to releasably hold an insert (300) for insertion into a bone,
b. at least one first recess (120) for receiving a first suture (400),
c. at least one second recess (130) for receiving a looped second suture (500), wherein the at least one first recess (120) and the at least one second recess (130) are configured such that the first suture (400) can be laid around the second suture (500) and the insert (300) thereby connecting the second suture (500) to the insert (300) when the insert (300) is held by the holding structure (110),
wherein the insert holding device (100) comprises a first surface (101), a second surface (102) facing away from the first surface (101), and a circumferential third surface (103) connecting the first surface (101) and the second surface (102), wherein the at least one first recess (120) and the at least one second recess (130) are open towards the first surface (101) and closed towards the second surface (102), and wherein the first recess (120) extends along a first axis (A₁) and the second recess (130) extends along a second axis (A₂) perpendicular to the first axis (A₁),
**characterized in that**
the insert holding device (100) comprises a groove (150) for inserting the second suture (500), wherein the groove (150) extends along said third surface (103) of the insert holding device (100) in a circumferential direction, and wherein said at least one second recess (130) is connected to said groove (150).

2. Insert holding device (100) according to claim 1, **characterized in that** the holding structure (110) comprises a slot (111) for receiving the insert (300), a first wall (112) and a second wall (113) opposing the first wall, wherein the slot (111) is delimited by the first wall (112) and the second wall (113), and wherein the first wall (112) and the second wall (113) are adapted to releasably hold the insert (300) within the slot (111) by static friction.

3. Suture holding device (200) for connecting a suture (500) to an insert (300) for insertion into a bone, comprising
a. a first recess (210) for inserting a flexible graft (900),
b. a holding structure (220) for positioning a looped second suture (500) across said first recess (210), wherein
c. the first recess (210) is configured such that an insert (300) for insertion into a bone held by the holding structure (110) of an insert holding device (100) can be positioned in the first recess (210) and a first suture (400) can be laid around the second suture (500) and the insert (300), thereby connecting the second suture (500) to the insert (300),
wherein the suture holding device (200) is formed from a flat sheet of material arranged in a plane defined by a first axis (A₁) and a second axis (A₂), wherein the suture holding device (200) comprises a front surface (201) and a back surface (202) opposing the front surface (201), and wherein said first recess (210) is a circular-shaped central first recess (210), **characterized in that** said holding structure (220) comprises a first through-hole (224) and a second through-hole (225) arranged along the second axis (A₂) in line with the first recess (210), and
wherein the suture holding device (200) comprises a first notch (287) and a second notch (288) in opposing edges, wherein the first notch (287) and the second notch (288) are arranged along the first axis (A₁) in line with the first recess (210), and
wherein the suture holding device (200) can be combined with the insert holding device (100) according to one of the claims 1 to 2 by placing the suture holding device (200) on top of the insert holding device (100) such that at least an upper portion (104) of the insert holding device (100) is positioned in the first recess (210), such that the first axis (A₁) of the insert holding device (100) is parallel to the first axis (A₁) of the suture holding device (200), and the second axis (A₂) of the insert holding device (100) is parallel to the second axis (A₂) of the suture holding device (200).

4. Suture holding device (200) according to claim 3, **characterized in that** the suture holding device (200) further comprises a first hole (281), a second hole (282), a third hole (283), a fourth hole (284), and a fifth hole (285) arranged essentially along a semi-circle around the first recess (210).

5. Suture holding device (200) according to claim 3 or 4, **characterized in that** a third notch (291) and a fourth notch (292) are provided in a further edge of the suture holding device (200), wherein a flap (290) is positioned between the third notch (291) and the fourth notch (292).

6. Kit of parts comprising an insert holding device (100) according to one of the claims 1 to 2 and a suture holding device (200) according to one of the claims 3 to 5, wherein the first recess (210) of said suture holding device (200) is configured such that an insert (300) for insertion into a bone held by the holding structure (110) of said insert holding device (100) can be positioned in the first recess (210) of said suture holding device (200) and a first suture (400) can be laid around a second suture (500) and the insert (300), thereby connecting the second suture (500) to the insert (300), wherein the second suture (500) is positioned across the first recess (210) of the suture holding device (200) by the holding structure (220) of the suture holding device (200).

7. Method for connecting a suture (500) to an insert (300) for insertion into a bone, wherein
a. an insert holding device (100) according to one of the claims 1 to 2 is provided, wherein
b. an insert (300) for insertion into a bone is received by a holding structure (110) of the insert holding device (100), such that the holding structure (110) releasably holds the insert (300), and wherein
c. a first suture (400) is received by at least one first recess (120) of the insert holding device (100), and wherein
d. a looped second suture (500) is received by at least one second recess (130) of the insert holding device (100), and wherein
e. the first suture (400) is laid around the second suture (500) and the insert (300) held by the holding structure (110) of the insert holding device (100), thereby connecting the second suture (500) to the insert (300) and wherein,
f. the second suture (500) is held by a holding structure (220) of a suture holding device (200) according to one of the claims 3 to 5 wherein the second suture (500) is positioned across a first recess (210) of the suture holding device (200), and wherein
g. the insert (300) held by the holding structure (110) of the insert holding device (100) is positioned in said first recess (210) of the suture holding device (200), and wherein
h. the first suture (400) is laid around the second suture (500) held by the holding structure (220) of the suture holding device (200) and the insert (300) held by the holding structure (110) of the insert holding device (100), thereby connecting the second suture (500) to the insert (300).

8. Method according to claim 7, wherein the insert holding device (100) is removed from the suture holding device (200), and wherein a flexible graft (900) is inserted into the first recess (210) of the suture holding device (200) after removing the insert holding device (100) from the suture holding device (200), and wherein the flexible graft (900) is connected to the second suture (500), thereby connecting the flexible graft (900) to the insert (300).

## Patentansprüche

1. Einsatzhaltevorrichtung (100) zum Verbinden einer Naht (500) mit einem Einsatz (300) zum Einsetzen in einen Knochen, umfassend:
a. eine Haltestruktur (110), die dazu eingerichtet ist, einen Einsatz (300) zum Einsetzen in einen Knochen lösbar zu halten,
b. mindestens eine erste Aussparung (120) zur Aufnahme einer ersten Naht (400),
c. mindestens eine zweite Aussparung (130) zur Aufnahme einer geschlungenen zweiten Naht (500), wobei die mindestens eine erste Aussparung (120) und die mindestens eine zweite Aussparung (130) so eingerichtet sind, dass die erste Naht (400) um die zweite Naht (500) und den Einsatz (300) gelegt werden kann, wodurch die zweite Naht (500) mit dem Einsatz (300) verbunden wird, wenn der Einsatz (300) durch die Haltestruktur (110) gehalten wird,
wobei die Einsatzhaltevorrichtung (100) eine erste Oberfläche (101), eine zweite Oberfläche (102), die der ersten Oberfläche (101) abgewandt ist, und eine umlaufende dritte Oberfläche (103) aufweist, die die erste Oberfläche (101) und die zweite Oberfläche (102) verbindet, wobei die mindestens eine erste Aussparung (120) und die mindestens eine zweite Aussparung (130) zu der ersten Oberfläche (101) hin offen und zu der zweiten Oberfläche (102) hin geschlossen sind, und wobei sich die erste Aussparung (120) entlang einer ersten Achse (A₁) erstreckt und die zweite Aussparung (130) sich entlang einer zweiten Achse (A₂) senkrecht zu der ersten Achse (A₁) erstreckt,
**dadurch gekennzeichnet, dass**
die Einsatzhaltevorrichtung (100) eine Nut (150) zum Einführen der zweiten Naht (500) aufweist, wobei sich die Nut (150) in einer Umfangsrichtung entlang der dritten Oberfläche (103) der Einsatzhaltevorrichtung (100) erstreckt, und wobei die mindestens eine zweite Aussparung (130) mit der Nut (150) verbunden ist.

2. Einsatzhaltevorrichtung (100) nach Anspruch 1, **dadurch gekennzeichnet, dass** die Haltestruktur (110) einen Schlitz (111) zur Aufnahme des Einsatzes (300), eine erste Wand (112) und eine der ersten Wand gegenüberliegende zweite Wand (113) aufweist, wobei der Schlitz (111) durch die erste Wand (112) und die zweite Wand (113) begrenzt ist, und wobei die erste Wand (112) und die zweite Wand (113) dazu angepasst sind, den Einsatz (300) durch Haftreibung lösbar in dem Schlitz (111) zu halten.

3. Nahthaltevorrichtung (200) zum Verbinden einer Naht (500) mit einem Einsatz (300) zum Einsetzen in einen Knochen, aufweisend
a. eine erste Aussparung (210) zum Einsetzen eines flexiblen Transplantats (900),
b. eine Haltestruktur (220) zum Positionieren einer geschlungenen zweiten Naht (500) über der ersten Aussparung (210), wobei
c. die erste Aussparung (210) so eingerichtet ist, dass ein Einsatz (300) zum Einsetzen in einen Knochen, der von der Haltestruktur (110) einer Einsatzhaltevorrichtung (100) gehalten wird, in der ersten Aussparung (210) positioniert werden kann und eine erste Naht (400) um die zweite Naht (500) und den Einsatz (300) gelegt werden kann, wodurch die zweite Naht (500) mit dem Einsatz (300) verbunden wird,
wobei die Nahthaltevorrichtung (200) aus einem flachen Materialblatt gebildet ist, das in einer Ebene angeordnet ist, die durch eine erste Achse (A₁) und eine zweite Achse (A₂) definiert ist, wobei die Nahthaltevorrichtung (200) eine Vorderfläche (201) und eine der Vorderfläche (201) gegenüberliegende Rückfläche (202) aufweist, und wobei die erste Aussparung (210) eine kreisförmige zentrale erste Aussparung (210) ist, **dadurch gekennzeichnet, dass**
die Haltestruktur (220) ein erstes Durchgangsloch (224) und ein zweites Durchgangsloch (225) aufweist, die entlang der zweiten Achse (A₂) in einer Linie mit der ersten Aussparung (210) angeordnet sind, und wobei die Nahthaltevorrichtung (200) eine erste Kerbe (287) und eine zweite Kerbe (288) in gegenüberliegenden Kanten aufweist, wobei die erste Kerbe (287) und die zweite Kerbe (288) entlang der ersten Achse (A₁) in einer Linie mit der ersten Aussparung (210) angeordnet sind, und wobei die Nahthaltevorrichtung (200) mit der Einsatzhaltevorrichtung (100) nach einem der Ansprüche 1 bis 2 kombiniert werden kann, indem die Nahthaltevorrichtung (200) so auf der Einsatzhaltevorrichtung (100) platziert wird, dass mindestens ein oberer Abschnitt (104) der Einsatzhaltevorrichtung (100) in der ersten Aussparung (210) positioniert ist, derart, dass die erste Achse (A₁) der Einsatzhaltevorrichtung (100) parallel zur ersten Achse (A₁) der Nahthaltevorrichtung (200) und die zweite Achse (A₂) der Einsatzhaltevorrichtung (100) parallel zur zweiten Achse (A₂) der Nahthaltevorrichtung (200) ist.

4. Nahthaltevorrichtung (200) nach Anspruch 3, **dadurch gekennzeichnet, dass** die Nahthaltevorrichtung (200) weiterhin ein erstes Loch (281), ein zweites Loch (282), ein drittes Loch (283), ein viertes Loch (284) und ein fünftes Loch (285) aufweist, die im Wesentlichen entlang eines Halbkreises um die erste Aussparung (210) herum angeordnet sind.

5. Nahthaltevorrichtung (200) nach Anspruch 3 oder 4, **dadurch gekennzeichnet, dass** eine dritte Kerbe (291) und eine vierte Kerbe (292) in einer weiteren Kante der Nahthaltevorrichtung (200) vorgesehen sind, wobei eine Lasche (290) zwischen der dritten Kerbe (291) und der vierten Kerbe (292) positioniert ist.

6. Teilesatz aufweisend eine Einsatzhaltevorrichtung (100) nach einem der Ansprüche 1 bis 2 und eine Nahthaltevorrichtung (200) nach einem der Ansprüche 3 bis 5, wobei die erste Aussparung (210) der Nahthaltevorrichtung (200) so eingerichtet ist, dass ein Einsatz (300) zum Einsetzen in einen Knochen, der von der Haltestruktur (110) der Einsatzhaltevorrichtung (100) gehalten wird, in der ersten Aussparung (210) der Nahthaltevorrichtung (200) positioniert werden kann und eine erste Naht (400) um eine zweite Naht (500) und den Einsatz (300) gelegt werden kann, wodurch die zweite Naht (500) mit dem Einsatz (300) verbunden wird, wobei die zweite Naht (500) durch die Haltestruktur (220) der Nahthaltevorrichtung (200) über der ersten Aussparung (210) der Nahthaltevorrichtung (200) positioniert wird.

7. Verfahren zum Verbinden einer Naht (500) mit einem Einsatz (300) zum Einsetzen in einen Knochen, wobei
a. eine Einsatzhaltevorrichtung (100) nach einem der Ansprüche 1 bis 2 zur Verfügung gestellt wird, und wobei
b. ein Einsatz (300) zum Einsetzen in einen Knochen von einer Haltestruktur (110) der Einsatzhaltevorrichtung (100) aufgenommen wird, so dass die Haltestruktur (110) den Einsatz (300) lösbar hält, und wobei
c. eine erste Naht (400) von mindestens einer ersten Aussparung (120) der Einsatzhaltevorrichtung (100) aufgenommen wird, und wobei
d. eine geschlungene zweite Naht (500) von mindestens einer zweiten Aussparung (130) der Einsatzhaltevorrichtung (100) aufgenommen wird, und wobei
e. die erste Naht (400) um die zweite Naht (500) und den Einsatz (300) herum gelegt wird, der von der Haltestruktur (110) der Einsatzhaltevorrichtung (100) gehalten wird, wodurch die zweite Naht (500) mit dem Einsatz (300) verbunden wird, und wobei,
f. die zweite Naht (500) durch eine Haltestruktur (220) einer Nahthaltevorrichtung (200) gemäß einem der Ansprüche 3 bis 5 gehalten wird,
wobei die zweite Naht (500) über einer ersten Aussparung (210) der Nahthaltevorrichtung (200) positioniert wird, und wobei
g. der Einsatz (300), der durch die Haltestruktur (110) der Einsatzhaltevorrichtung (100) gehalten wird, in der ersten Aussparung (210) der Nahthaltevorrichtung (200) positioniert wird, und wobei
h. die erste Naht (400) um die zweite Naht (500), die von der Haltestruktur (220) der Nahthaltevorrichtung (200) gehalten wird, und den Einsatz (300), der von der Haltestruktur (110) der Einsatzhaltevorrichtung (100) gehalten wird, herum gelegt wird, wodurch die zweite Naht (500) mit dem Einsatz (300) verbunden wird.

8. Verfahren nach Anspruch 7, wobei die Einsatzhaltevorrichtung (100) von der Nahthaltevorrichtung (200) entfernt wird, und wobei ein flexibles Transplantat (900) in die erste Aussparung (210) der Nahthaltevorrichtung (200) eingesetzt wird, nachdem die Einsatzhaltevorrichtung (100) von der Nahthaltevorrichtung (200) entfernt wurde, und wobei das flexible Transplantat (900) mit der zweiten Naht (500) verbunden wird, wodurch das flexible Transplantat (900) mit dem Einsatz (300) verbunden wird.

## Revendications

1. Dispositif de maintien d'insert (100) pour relier une suture (500) à un insert (300) destiné à être inséré dans un os, comportant
a. une structure de maintien (110) conçue pour maintenir de manière libérable un insert (300) destiné à être inséré dans un os,
b. au moins un premier évidement (120) destiné à recevoir une première suture (400),
c. au moins un second évidement (130) destiné à recevoir une seconde suture (500) en boucle, dans lequel le au moins un premier évidement (120) et le au moins un second évidement (130) sont conçus de sorte que la première suture (400) puisse être déposée autour de la seconde suture (500) et de l'insert (300), reliant ainsi la seconde suture (500) à l'insert (300) lorsque l'insert (300) est maintenu par la structure de maintien (110),
dans lequel le dispositif de maintien d'insert (100) comporte une première surface (101), une deuxième surface (102) face opposée à la première surface (101) et une troisième surface (103) circonférentielle reliant la première surface (101) à la deuxième surface (102), dans lequel le au moins un premier évidement (120) et le au moins un second évidement (130) sont ouverts vers la première surface (101) et fermés vers la deuxième surface (102) et dans lequel le premier évidement (120) s'étend le long d'un premier axe (A₁) et le second évidement (130) s'étend le long d'un second axe (A₂) perpendiculaire au premier axe (A₁),
**caractérisé en ce que**
le dispositif de maintien d'insert (100) comporte une rainure (150) pour l'insertion de la seconde suture (500), dans lequel la rainure (150) s'étend le long de ladite troisième surface (103) du dispositif de maintien d'insert (100) dans une direction circonférentielle et dans lequel ledit au moins un second évidement (130) est relié à ladite rainure (150).

2. Dispositif de maintien d'insert (100) selon la revendication 1, **caractérisé en ce que** la structure de maintien (110) comporte une fente (111) pour recevoir l'insert (300), une première paroi (112) et une seconde paroi (113) opposée à la première paroi, dans lequel la fente (111) est délimitée par la première paroi (112) et la seconde paroi (113) et dans lequel la première paroi (112) et la seconde paroi (113) sont adaptées pour maintenir l'insert (300) de façon libérable au sein de la fente (111) par frottement statique.

3. Dispositif de maintien de suture (200) pour relier une suture (500) à un insert (300) destiné à être inséré dans un os, comportant
a. un premier évidement (210) pour insérer une greffe souple (900),
b. une structure de maintien (220) pour positionner une seconde suture (500) en boucle à travers ledit premier évidement (210), dans lequel
c. le premier évidement (210) est conçu de sorte qu'un insert (300) destiné à être inséré dans un os maintenu par la structure de maintien (110) d'un dispositif de maintien d'insert (100) puisse être positionné dans le premier évidement (210) et qu'une première suture (400) puisse être déposée autour de la seconde suture (500) et de l'insert (300), reliant ainsi la seconde suture (500) à l'insert (300),
dans lequel le dispositif de maintien de suture (200) est formé à partir d'une feuille plate de matériau agencée dans un plan défini par un premier axe (A₁) et un second axe (A₂), dans lequel le dispositif de maintien de suture (200) comporte une surface avant (201) et une surface arrière (202) opposée à la surface avant (201) et dans lequel ledit premier évidement (210) est un premier évidement central (210) de forme circulaire, **caractérisé en ce que** ladite structure de maintien (220) comporte un premier trou traversant (224) et un second trou traversant (225) agencés le long du second axe (A₂) en ligne avec le premier évidement (210) et dans lequel le dispositif de maintien de suture (200) comporte une première encoche (287) et une deuxième encoche (288) dans des bords opposés, dans lequel la première encoche (287) et la deuxième encoche (288) sont agencées le long du premier axe (A₁) en ligne avec le premier évidement (210) et
dans lequel le dispositif de maintien de suture (200) peut être associé au dispositif de maintien d'insert (100) selon l'une des revendications 1 à 2 par placement du dispositif de maintien de suture (200) sur le dessus du dispositif de maintien d'insert (100), de sorte qu'au moins une portion supérieure (104) du dispositif de maintien d'insert (100) soit positionnée dans le premier évidement (210), de sorte que le premier axe (A₁) du dispositif de maintien d'insert (100) soit parallèle au premier axe (A₁) du dispositif de maintien de suture (200) et que le second axe (A₂) du dispositif de maintien d'insert (100) soit parallèle au second axe (A₂) du dispositif de maintien de suture (200).

4. Dispositif de maintien de suture (200) selon la revendication 3, **caractérisé en ce que** le dispositif de maintien de suture (200) comporte en outre un premier trou (281), un deuxième trou (282), un troisième trou (283), un quatrième trou (284) et un cinquième trou (285) agencés essentiellement le long d'un demi-cercle autour du premier évidement (210).

5. Dispositif de maintien de suture (200) selon la revendication 3 ou 4, **caractérisé en ce qu'**une troisième encoche (291) et une quatrième encoche (292) sont prévues dans un bord plus avancé du dispositif de maintien de suture (200), dans lequel un rabat (290) est positionné entre la troisième encoche (291) et la quatrième encoche (292).

6. Kit de pièces comportant un dispositif de maintien d'insert (100) selon l'une des revendications 1 à 2 et un dispositif de maintien de suture (200) selon l'une des revendications 3 à 5, dans lequel le premier évidement (210) dudit dispositif de maintien de suture (200) est conçu de sorte qu'un insert (300) destiné à être inséré dans un os maintenu par la structure de maintien (110) dudit dispositif de maintien d'insert (100) puisse être positionné dans le premier évidement (210) dudit dispositif de maintien de suture (200) et qu'une première suture (400) puisse être déposée autour d'une seconde suture (500) et de l'insert (300), reliant ainsi la seconde suture (500) à l'insert (300), dans lequel la seconde suture (500) est positionnée à travers le premier évidement (210) du dispositif de maintien de suture (200) par la structure de maintien (220) du dispositif de maintien de suture (200).

7. Procédé de liaison d'une suture (500) à un insert (300) destiné à être inséré dans un os, dans lequel
a. un dispositif de maintien d'insert (100) selon l'une des revendications 1 à 2 est prévu, dans lequel
b. un insert (300) destiné à être inséré dans un os est reçu par une structure de maintien (110) du dispositif de maintien d'insert (100), de sorte que la structure de maintien (110) maintienne l'insert (300) de manière libérable et dans lequel
c. une première suture (400) est reçue par au moins un premier évidement (120) du dispositif de maintien d'insert (100) et dans lequel
d. une seconde suture (500) en boucle est reçue par au moins un second évidement (130) du dispositif de maintien d'insert (100) et dans lequel
e. la première suture (400) est déposée autour de la seconde suture (500) et l'insert (300) maintenu par la structure de maintien (110) du dispositif de maintien d'insert (100), reliant ainsi la seconde suture (500) à l'insert (300), et dans lequel
f. la seconde suture (500) est maintenue par une structure de maintien (220) d'un dispositif de maintien de suture (200) selon l'une des revendications 3 à 5, dans lequel la seconde suture (500) est positionnée à travers un premier évidement (210) du dispositif de maintien de suture (200) et dans lequel
g. l'insert (300) maintenu par la structure de maintien (110) du dispositif de maintien d'insert (100) est positionné dans ledit premier évidement (210) du dispositif de maintien de suture (200) et dans lequel
h. la première suture (400) est déposée autour de la seconde suture (500) maintenue par la structure de maintien (220) du dispositif de maintien de suture (200) et de l'insert (300) maintenu par la structure de maintien (110) du dispositif de maintien d'insert (100), ce qui relie la seconde suture (500) à l'insert (300).

8. Procédé selon la revendication 7, dans lequel le dispositif de maintien d'insert (100) est retiré du dispositif de maintien de suture (200) et dans lequel une greffe souple (900) est insérée dans le premier évidement (210) du dispositif de maintien de suture (200) après retrait du dispositif de maintien d'insert (100) à partir du dispositif de maintien de suture (200) et dans lequel la greffe souple (900) est reliée à la seconde suture (500), ce qui relie la greffe souple (900) à l'insert (300).
